(19) Europäisches Patentamt
European Patent Office
Office européen des brevets

(11) **EP 4 616 818 A1**

(12) **EUROPEAN PATENT APPLICATION**

(43) Date of publication:
**17.09.2025 Bulletin 2025/38**

(21) Application number: **25160538.2**

(22) Date of filing: **27.02.2025**

(51) International Patent Classification (IPC):
**A61B 18/12** $^{(2006.01)}$     **A61B 18/00** $^{(2006.01)}$

(52) Cooperative Patent Classification (CPC):
**A61B 18/1206;** A61B 18/1233; A61B 2018/00648;
A61B 2018/00678; A61B 2018/00702;
A61B 2018/00779; A61B 2018/00827;
A61B 2018/00869; A61B 2018/00892

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.03.2024   US 202463564118 P**

(71) Applicant: **Olympus Winter & Ibe GmbH
22045 Hamburg (DE)**

(72) Inventors:
• **DIJKSTRA, Jelle**
  **12205 Berlin (DE)**
• **RAMIN, Daniel**
  **14558 Nuthetal (DE)**
• **FÄHSING, Thomas**
  **12305 Berlin (DE)**

(74) Representative: **Eisenführ Speiser
Patentanwälte Rechtsanwälte PartGmbB
Postfach 10 60 78
28060 Bremen (DE)**

(54) **METHOD FOR VERIFYING A POWER RECORDING OF AN RF GENERATOR OF AN ELECTROSURGICAL INSTRUMENT AND CORRESPONDING RF GENERATOR**

(57) Method for verifying a power recording of an RF generator to control at least one electrosurgical instrument, wherein the RF generator has an inverter for generating an RF signal, and the inverter via an Inverter input is supplied with electrical current and via an Inverter output emits an RF signal with a voltage value and a current value for driving an electrosurgical instrument, comprising the steps recording of an output power delivered via the RF signal by directly measuring the RF signal by means of an RF power acquisition device, as directly recorded output power, recording of a comparative power based on a power measured on the input side of the inverter and verifying the functioning of the RF power acquisition device by comparing the recorded comparative power with the directly recorded output power.

Fig. 1

**Description**

The invention is directed to a method for verifying a power recording of an RF generator to control at least one electrosurgical instrument. The invention is also directed to an RF generator using such method.

For controlling an electrosurgical instrument, an RF generator is used which supplies the electrosurgical instrument with an RF signal having a particular power. The power depends on the particular task to be currently performed by the electrosurgical instrument.

As such electrosurgical instrument is applied to a human body, safety issues are quite important. One safety issue is to make sure that the correct power, i.e. the correct amount of power, is supplied by the RF generator. In order to ensure this, there must be a redundant system for evaluating the supplied power. The supplied power can be used for a feedback control and accordingly the control also depends on the determined power which is supplied by the RF generator.

For recording, i.e. estimating or measuring the output power of the RF generator which is supplied to the electrosurgical instrument, voltage and current of the output signal can be measured and the corresponding output power can be calculated. Determining the power in this way is not simple due to the high frequency and high voltage of the signal. For this calculation, a phase angle between voltage and current is important and thus a good accuracy of the phase angle for such high frequency signal is needed. However, this can be done by a well-known FPGA (Field Programmable Gate Array).

In order to have a redundant recordal of the output power, two similar systems may be implemented. I. e. 2 FPGA can be used to determine the output power and the determined values of both systems can be compared in order to verify if the determined output power is correct. If there is a significant difference between these two output powers determined this way, there must be an error. Problems can occur due to identical or similar methods or systems for measuring the output parameter. Hardware, an FPGA code and as well software might have a design error which could be in both paths and could lead to the same error behavior. Accordingly, a comparison of such two signals having the same error could result in not recognizing such error. It is also important to mention that such way of measuring the RF current and RF voltage can be complicated and costly.

It was also found that any additional sensors, i.e. a redundant sensor, may cause an increased kind of leakage current which shall however be reduced rather than increased.

Accordingly, such redundant system explained above has the following disadvantages. Such system may be prone to errors in view of design and systematical errors. The system is costly due to the redundant systems. Also higher leakage currents occur due to bypassing the corresponding path.

Accordingly, the object of the present invention is to suggest at least one solution addressing at least one of the above identified drawbacks. In particular, a solution shall be suggested for verifying the recordal of the output power of an RF generator supplying an electrosurgical instrument that avoids systematical errors, reduces costs and avoids an increase of leakage currents. There shall at least be suggested an alternative with respect to so far known solutions.

According to the invention, a method is suggested according to claim 1. Such method is thus directed for verifying a power recording of an RF generator to control at least one electrosurgical instrument. Such RF generator has an inverter for generating an RF signal. The inverter is supplied with electrical current via an inverter input. There might be a power supply for supplying the inverter with said electrical current. Such power supply may be part of the RF generator or it may be an external device.

Via an inverter output, the inverter emits an RF signal with a voltage value and a current value for driving the electrosurgical instrument. Accordingly, the inverter changes the input signal, which could be a DC input current, into such RF signal. The RF signal is thus driving the electrosurgical instrument and according to the actual needs of the electrosurgical instrument the voltage value and/or the current value can be set. This is also done such that a particular power value, i.e. of the recorded power, is met.

An output power is thus delivered via the RF signal and such output power is recorded by directly measuring the RF signal by means of an RF power acquisition device as directly recorded output power. Such power acquisition device may be an FPGA as explained above. The RF signal emitted by the inverter output can also be understood as an RF output signal. Accordingly, this RF output signal is measured as explained. Accordingly, the power acquisition device is thus measuring at the inverter output and it is designed to measure an RF signal and based on that estimate the power that is provided by such RF signal.

There is also recorded a comparative power based on a power measured on the input side of the inverter, i.e. at the inverter input. In particular, the power supplied by a power supply connected to the inverter input can be measured. As explained, the power supply at the inverter input could be using a DC current and such DC current would be measured, in addition a corresponding voltage is measured, in order to record the power at the input side of the inverter. Even if the power at the input side would not be supplied by a DC current, it could be supplied by a low frequency signal of 50 Hz or 60 Hz. For such DC signals or signals having such low frequency inexpensive devices for measuring the power are well-known and available. Such devices can be used and thus the comparative power can be measured in a fairly inexpensive way.

Based on that it is suggested to verify the functioning of the RF power acquisition device by comparing the recorded comparative power with the directly recorded output power.

In this way, a solution is suggested that avoids an additional redundant measuring system which is costly and possibly complicated. That solution also avoids systematical errors as the recording of the comparative power is systematically different from the directly recording of the output power at the inverter output. Additional leakage currents are also avoided as the suggested solution makes the measurement of the comparative power at the input side of the inverter and thus avoids such leakage currents at the inverter output.

According to one aspect, it is suggested that on the inverter input an input power is recorded and the comparative power is determined from the recorded input power minus a power dissipation. It was thus realized that the output power must in general correspond to the input power minus any dissipation power. Accordingly, it is suggested to determine the comparative power not only based on the power measurement at the input of the inverter, but also on the power dissipation. It was found that such power dissipation can be calculated with a good accuracy and that this can be done without costly measurement equipment. Particular values which are influencing the dissipative power are known or can be evaluated offline. Corresponding parameters are explained below with respect to possible aspects of the invention.

Accordingly, the comparative power can be determined with high accuracy but without costly measurement equipment, if the dissipative power is also considered. In this way, a good redundancy can be achieved. There can thus be suggested a good and reliable solution.

According to one aspect, the functioning of the RF power acquisition device is verified if an absolute value of a difference between the recorded comparative power and the directly recorded output power, is below a predetermined verification threshold value. The difference is thus determined by the comparison of the recorded comparative power with the directly recorded output power. In particular, the verification threshold value is in a range between 5% and 30% and in particular 10% and 20% of the directly recorded output power.

In this way, there is a clear criterion for deciding whether a correct functioning of the RF power acquisition device may be assumed or whether not. In particular, it is suggested using this verification threshold value as a relative threshold value, i.e. relative to the amplitude of the directly recorded output power.

In particular, it was found that a value of 5% of the directly recorded output power would not indicate a malfunction, as in particular the determining of the power dissipation could have a difference in this range just due to any inaccuracy. Accordingly, if such verification threshold value would be chosen to be too small, i.e. below 5%, the problem of a false alarm would arise. On the other hand, an upper value for the verification threshold value of 30% seems to be quite vague. However, it should be kept in mind that in case of a malfunction a very big difference between the directly recorded output power and the comparative output power will occur. Based on these findings, the lower value for the verification threshold value can be chosen to be 10% as that is still an acceptable accuracy, and the upper value can be chosen to be 20% or 25% of the directly recorded output power as that would make it possible to identify malfunctions that appear without a too big discrepancy. In other words, a malfunction having only a discrepancy of a bit more than 20% or a bit more than 25% could still be identified.

According to one aspect, the power dissipation is determined depending on at least one loss parameter. Possible loss parameters will be described below. In particular, it was found that such loss parameters are known or can be determined and can be used to evaluate the power dissipation and thus facilitate calculating the comparative power.

According to one aspect, the loss parameter can be a degree of modulation of the inverter. The degree of modulation of the inverter can be defined as the ratio of the amplitude of the modulated signal to the amplitude of the D/C voltage of the inverter, i.e. of the intermediate circuit. The inverter might control a modulated current signal but this current signal also has a voltage amplitude. This voltage amplitude, in particular the peak value of the voltage signal, is thus considered. The ratio of such peak value to the amplitude of the D/C voltage of the intermediate circuit can thus be taken as the degree of modulation which can also be called modulation degree.

Such modulation degree is thus in the range of 0 to 1. In particular, a high modulation degree indicates an efficient operating point. The less efficient the operating point is, the higher is the relative power dissipation. However, the most efficient operating point is not for a modulation degree of 1. It is more in the range between 0.8 and 0.9.

Accordingly, the modulation degree is always well-known as the exact operation of the inverter is well-known. Accordingly, any power dissipation due to the modulation can be estimated with a good accuracy.

According to one aspect, a selected RF mode can be used as a loss parameter. The RF mode, i.e. the high frequency mode, can indicate a characteristic of the selected mode of the inverter.

RF mode means the setting or the purpose of the planned or selected operation, such as cutting mode, coagulation mode or other modes. It was found that there are continuous modes, or continuous sine oscillations and modulated oscillations. Modulated oscillations could be oscillation that are interrupted, e.g. 2 oscillations with 350 kHz, followed by a pause, then again 2 oscillations. A Forced Coag mode would be an example. This then repeats itself at approx. 18 kHz, to give one example. Here too, modulation always means amplitude modulation, i.e. modulating the voltage in the intermediate circuit or using a PWM.

It was found that also depending on the selected RF mode the power dissipation may vary and as the selected RF mode is well-known, the corresponding power dissipation can also be calculated with a high accuracy if the selected RF mode is thus considered.

The ambient temperature of the inverter can also be used as a loss parameter. Due to the ambient temperature of the inverter, which could be measured by using a temperature sensor placed at the casing of the inverter, or placed in a distance from the inverter of less than 10 m or in particular less than 1 m. If the inverter is operated in a room, a room temperature can used as the ambient temperature. It was found that such ambient temperature influences the temperature in the inverter and thus can lead to a higher power dissipation if the temperature is higher. It was also found that a high ambient temperature could result in a higher activity of a cooling vent which will result in a higher power consumed by such cooling vent. Accordingly, the power needed for such cooling vent could be considered indirectly by considering the ambient temperature.

According to one aspect, a component temperature of a component of the inverter can be used as a loss parameter. Such component temperature can in particular be the temperature of a semiconductor and/or of a circuitry carrying a couple of semiconductors, in particular a circuitry comprising semiconductor switches for generating a pulsed signal for generating the RF-signal. Such semiconductors are executing the most work with regard to transforming power and are dissipating power mainly as thermal power. Accordingly, the temperature of such component of the inverter, in particular, of such semiconductors or circuitry gives an indication of the dissipated power. Accordingly, the power dissipation can be calculated based on that with a high accuracy.

According to one aspect, a switching frequency of the inverter can be used as a loss parameter. It was found that such switching frequency can influence losses and is thus suggested to be considered. In particular, the switching frequency of the inverter, may influence a loss of a transformer and accordingly a loss of a transformer may be evaluated depending on the switching frequency. In particular a loss of a transformer as explained in the following paragraphs can be evaluated. According to one aspect, a loss of a transformer can be used as a loss parameter. Such transformer can be used to transform the RF-signal generated by the inverter to a higher voltage level needed by the connected electrosurgical instrument. Such loss of the transformer can be significant and thus can increase the accuracy of the calculated power dissipation if that transformer loss is considered.

The primary current is measured at the transformer in order to compare the current with the target current and, if necessary, actively counteract it, in particular having a state feedback, e.g. in the event of distortions due to resonances, which develop in particular with no load or a very high load.

In addition to the switching frequency, or as an alternative, a measured current and/or measured voltage, or other parameters, could be used to determine the loss of the transformer. It is also possible, to use a model, in particular a theoretical model.

According to one aspect, at least one loss parameter is recorded in advance, in particular before the inverter is put into operation, and assigned to a power dissipation and/or at least one loss parameter is recorded during operation of the inverter and assigned to a power dissipation.

Therefore, at least for the first feature explained to this aspect it was found that at least some of the loss parameters mentioned above can be considered and used for calculating the power dissipation. Some loss parameters do not change fast making it possible to consider these before the start of the inverter. Being recorded in advance, in particular before the inverter is put into operation, may in particular mean just before the operation. For example, a particular operation step is selected that may include the used surgical instrument and separately or depending on the used electrosurgical instrument a loss parameter such as the degree of modulation can be selected or the selected RF mode is also known. Such parameters can be recorded just before starting the operating, i.e. just before starting the inverter to modulate the particular signal.

On the other hand, an ambient temperature of the inverter can also be measured before which will not change within the next minutes. In this way, a particular consideration of such loss parameters is ensured even at the start of the operation. Thus, the redundant determination of the output current is ensured from the beginning.

In addition or alternatively, at least one loss parameter can be recorded during operation and that is in particular directed to loss parameters which might change during operation. In particular, it is suggested considering a component temperature during operation of the inverter. It was found that the component temperature in particular of semiconductors and even further of the mentioned circuitry may change quickly and change its amplitude during the operation, i.e. during a single operation task. Even operation tasks that might take only some seconds as for example 5 to 20 seconds, are long enough that a component temperature can change significantly. Accordingly as this was found, it is suggested recording the corresponding loss parameter during operation of the inverter and assign it to a power dissipation and thus to consider it when determining the comparative power.

According to one aspect, the inverter has a DC voltage input and to record the comparative power, the input power from a DC voltage and/or DC current measurement at the inverter input is detected. For this idea, the underlying structure comprises an inverter with a DC input and the AC output. The inverter thus receives a DC input power and it was found that such input power can be used to calculate the comparative power. Such DC power can easily be measured by measuring, according to one example, DC voltage and DC current simultaneously and calculating the power, in particular by multiplying the measured voltage and current. Such multiplication can easily be done if the underlying signal is each a DC signal. In this way, a good basis of the input power can be achieved with simple measurement equipment and still with a good accuracy. After further considering the dissipative power, a comparative power with good accuracy can be determined.

According to one aspect, the RF power acquisition device comprises an FPGA (Field Programmable Gate Array) connected via an isolating transformer to the inverter output in order to measure the RF signal. In combination with that, for measuring the power on the input side of the inverter, a DC measuring device is used. The DC measuring device could also be used if a FPGA is not used for measuring the RF signal at the inverter output.

However, the combination of the FPGA with the isolating transformer at the inverter output and the DC measuring device at the inverter input has particular advantages.

The FPGA connected via the isolating transformer to the inverter output can measure the output power quickly and with a high accuracy and usually also in a reliable way.

Accordingly, it was found that just for possibly identifying a malfunction of this measurement using the FPGA, the DC measuring device can be a not very costly but still very reliable measurement solution. Such solution might not be as accurate with respect to the output power as the used FPGA, but it was found that it can be accurate enough in order to verify whether the FPGA is working properly or if there is a malfunction of it.

Accordingly, the combination of the FPGA using the insulating transformer at the output side in combination with the DC measuring device at the input side results in the explained synergetic advantage.

It is also important to mention that the effort of using an FPGA connected via an insulating transformer to the inverter output is quite high and in particular quite costly. It was found that for ensuring the needed redundancy, the solution of using the DC measuring device at the input side can thus avoid to need two measuring solutions, each having an FPGA connected via an isolating transformer to the inverter output. Thus, this combination provides a good and reliable solution which is however not so costly.

In addition, the FPGA connected via an isolating transformer to the inverter on the one hand and the DC measuring device on the other hand are based on different measuring principles and accordingly systematic errors can be avoided. For example, if the FPGA has a logic malfunction, this could also be present in the other FPGA as well, leading to two false measurements which may however provide similar, but false measuring values. Comparing these two false measuring values might in that case not be adequate to identify the malfunction.

According to one aspect, a model is used for determining the comparative power and/or for determining the power dissipation. It was found that by using a model all significant elements and effects resulting in a power loss can be taken into account without much effort. Such model can get in particular all loss parameters as input and based on that the model can simply output the particular power dissipation.

Such model can be or included a transmission model of the inverter that determines the losses depending on certain modes of the inverter.

The model can be designed by using known circumstances such as a power dissipation of the inverter depending on the degree of modulation and/or depending on the selected high frequency mode. The power dissipation of components of the inverter, in particular of semiconductors, is often known, or at least a model of such semiconductors is known as semiconductors are usually designed using simulations which are based on a very precise model of the semiconductor. Such model or a simplified model based on that can be used.

In addition or alternatively, it is possible to measure the power dissipation while recording the explained loss parameters. This can be done offline before taking the RF generator in operation. It is also possible to use a complex simulation for testing the whole RF generator including an attached electrosurgical instrument and based on such complex simulation the behavior of the power dissipation depending on the power loss parameters can also be simulated. Based on that, a particular model for modeling the power dissipation can be designed.

It is also possible to constantly measure a power dissipation and based on that, design the model by online learning or at least verify the model by online learning.

Based on the power dissipation determined by use of such model, the comparative power can be calculated. However, calculating the comparative power can also be done by such model. In other words, the calculation of the comparative power can also be integrated into the functionality of said model. In that case, the model can directly output the comparative power. However, the power dissipation could also be outputted by such model just as an additional value which can be used to verify the correct operation of the model.

According to one aspect, an adaptive and/or self-learning estimation method and/or estimation model having estimation parameters is used to estimate the comparative power. Accordingly, it was found that the method for estimating the comparative power can be provided as an adaptive and/or self-learning estimation method resulting in more and more improved, in particular more and more accurate results. This method can be implemented in a model and thus the model can be adaptive and/or self-learning accordingly.

Such method or model has estimation parameters which are in particular used to estimate the power dissipation and based on that estimate or calculate the comparative power. This is also based on the power measured on the input side of the inverter. To give a simple example, one estimation parameter could be a factor to calculate part of the dissipation power by multiplying that factor with the recorded input-power. The factor could have a starting value of 0.1, i.e. 10%, and can be adapted during operation. The dissipation power can be calculated adding that part of the dissipation power to further parts of the dissipation power.

It was also found that the estimation of the comparative power, in particular the estimation of the power dissipation needed for this, is part of providing a redundant power measurement or redundant recordal of a power. I.e. the comparative power is redundant to a quite accurate power measurement, in particular, one that is using an FPGA as explained above. It was thus found that such measurement, i.e. the measurement or estimation of the directly recorded output power, can also be used for adaption and/or self-learning for the recording or estimation of the comparative power.

The estimation parameters can in particular be any of parameters describing a relationship between any of the loss parameters explained above. An estimation parameter can thus be a relationship between the degree of modulation of the inverter and a resulting power dissipation. It can also be the relationship between the selected RF mode and a resulting power dissipation. It can also be a relationship between an ambient temperature of the inverter and a resulting power dissipation. It can also be a relationship between a component temperature and a resulting power dissipation.

It is to be noted that the power dissipation considered for calculating the comparative power based on the power measured at the input side can be the sum of the explained resulting power dissipation resulting for each explained loss parameter. Of course, further power dissipation part could be added as well. In particular, there could be a residual power dissipation covering all power dissipation not mentioned and/or not considered. The loss of a transformer can also be added up to the power dissipation. Accordingly, the power dissipation used for calculating the comparative power based on the power measured at the input side can also be called overall power dissipation. In other words, the comparative power is determined from the recorded input power minus a such overall power dissipation.

One estimation parameter could be a relationship between any power dissipation parts explicitly considered and the residual power dissipation. For example, if the explicitly considered power dissipation parts sum up to 100 W but it was found that in fact the overall power dissipation is 110 W. For this example, the estimation parameter describing a relationship between the explicitly considered power dissipation which is summed up, to the residual power dissipation would be 0.1 according to this illustrative example. Alternatively, the estimation parameter could be a relationship between the explicitly considered power dissipation parts (summed up to 100 W) and the overall power dissipation (110 W in this example), and thus the estimation parameter would be 1.1 according to that example.

In particular, it is suggested that the comparative power is estimated based on initially set or previously adapted values of the estimation parameters. Accordingly, there are initially estimation parameters having default values when the device or the model or method is started for the first time. After that, the estimation values could be further adapted and thus the actual values of these estimation parameters are the basis for further adaption and/or self-learning.

Further, the estimated comparative power is compared with the directly recorded output power. If depending on the result of the comparison of the functioning of the RF generator is verified, i.e. if it was found that there is no malfunction, the directly recorded output power is identified as verified output power. Here, the underlying idea and finding is that the value of the directly recorded output power is assumed to be quite accurate, unless there was a malfunction. Accordingly, if it was identified that there is no malfunction, the value of the directly recorded output power is assumed to be quite accurate.

Accordingly, it is further suggested that the estimation parameters are adapted to align the estimated comparative power to the verified output power.

To explain with the above example and thus explain it only as an illustrative explanation with respect to only one estimation parameter, the estimation parameter for considering the residual power dissipation was 1.1. It calculated the explicitly considered 100 W to the overall power dissipation of 110 W. However, if it was found by comparing to the directly recorded output power that the overall power dissipation is in fact 120 W, said estimation parameter just mentioned could be changed from 1.1 to 1.2. It is however suggested not completely changing this parameter but letting it move towards this new value in an asymptotic way. Based on the above example, the value of 1.1 could just be amended by 10% of the total difference. Accordingly, the value would be changed from 1.1 to 1.11. If next time there would be again the same difference, it could further be amended from 1.11 to 1.12. This has a kind of filtering effect from the identified difference to the resulting adjustment of the estimation parameter.

According to the invention, there is also an RF generator suggested. Such RF generator is designed to control at least one electrosurgical instrument which is connected to the RF generator. The electrosurgical instrument can be part of the generator or can be a separate element. However, the particular functioning corresponds to a situation when the electrosurgical instrument is connected. It is also possible that different electrosurgical instruments are connected, either in parallel or one after another at the same or different output plug of the RF generator.

The RF generator is adapted to verify a power recording of the RF generator. The recorded power is the power provided to the electrosurgical instrument connected to the RF generator when being operated.

The RF generator has an inverter for generating an RF signal. This RF signal can be fed to the electrosurgical instrument to supply the electrosurgical instrument with the energy and with the particular signal to operate the electrosurgical instrument.

When in operation, the inverter is supplied with electrical current via an inverter input, and via an inverter output the inverter emits an RF signal with a voltage value and a current value for driving the electrosurgical instrument. The RF generator is also adapted to execute the following steps.

According to one step, the RF generator is recording an output power delivered via the RF signal by directly measuring the RF signal by means of an RF power acquisition device. This output power is referred to as directly recorded output power. A further step is recording a comparative power and that recording is based on a power measured on the input side of the inverter. Even further one step is verifying the functioning of the RF power acquisition device by comparing the recorded comparative power with the directly recorded output power.

The RF generator can be adapted to do this by having an RF controller which is adapted for executing and/or controlling the steps for verifying the power recording. The steps, in particular according to any of the aspects mentioned before, can be executed using a computer program being implemented of the RF controller, Of course, the RF controller may also execute further functions, in particular controlling the RF generator in order to provide the RF signal for operating the connected electrosurgical instrument.

According to one aspect, the RF generator and in particular its RF controller is adapted for performing at least one method according to any one aspect explained above with respect to the method for verifying a power recording.

The invention will now be described by way of example based on an embodiment referring to the accompanying figures.

Figure 1 shows an RF generator to control at least one electrosurgical instrument in a schematical view.

Figure 2 shows a diagram to illustrate input and output power with respect to dissipation power.

Figure 3 shows a simplified flow chart for a method according to at least one aspect.

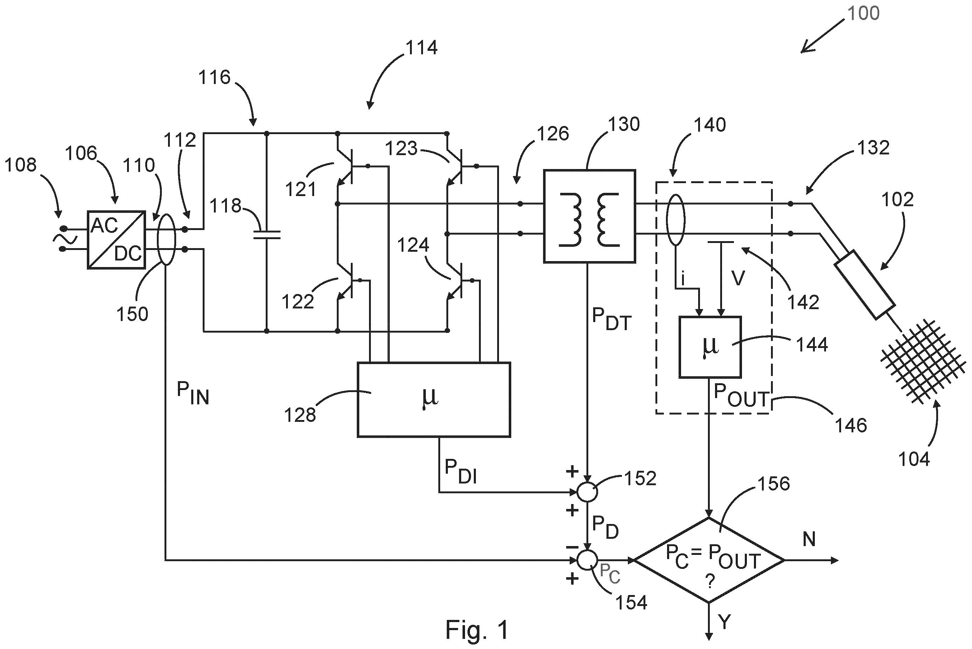

**Figure 1** shows an RF generator 100 in a schematical view having an electro surgical instrument 102 connected for operating at a tissue 104 just shown in a symbolic way. An RF generator to control at least one electro surgical instrument is in general quite complex and comprises a lot of elements. However in the shown RF generator 100 only some basic elements are explained which are useful for understanding the invention.

The RF generator 100 comprises a DC-supply 106 for supplying the RF generator 100 with DC power. The DC-supply 106 may be connected at its supply input 108 with a regular power supply grid supplying a 110 V or 230 V Voltage.

The DC-supply 106 is basically rectifying such input voltage received at its supply input 108 into a DC signal outputted at its supply output 110. The DC-supply 106 may as well comprise means for changing the voltage amplitude at its supply output 110. The supply output 110 is connected to a DC-input 112 of an inverter circuit 114. The inverter circuit 114 has a DC intermediate circuit 116 having an intermediate capacitor 118. The inverter 114 comprises four semiconductor switches 121 - 124 connected to the intermediate circuit 116 for generating an AC-output signal at an AC-output 126 of the inverter circuit 114. Such generating of the AC-output signal is based on a DC-voltage in the DC intermediate circuit 116. Said semiconductor switches 121 - 124 could be IGBTs. The semiconductor switches 121 - 124 generate a RF-output signal by pulse - modulation such as a PWM. However a different circuitry and different method for modulation could be used.

For controlling the modulation the four semiconductor switches 121 - 124 are controlled by an inverter controller 128. The inverter control 128 is accordingly connected to each of this semiconductor switches 121 - 124 and is thus, by controlling the semiconductor switches, also having any information related with that modulation such as a modulation frequency and a degree of modulation. The inverter control 128 may also be aware of temperatures of the inverter circuit or even in detail temperatures of each of the four semiconductor switches 121 - 124. For that purpose temperature sensors may be provided which are not shown in figure 1 in order to keep the schematical structure simple.

Connected to the AC-output 126 of the inverter circuit is an RF-transformer 130. The RF-transformer 130 transforms the AC-signal of the AC-output 126 to a higher voltage to provide this RF-signal of higher voltage at the generator output 132. Connected to this generator output 132 is the electro surgical 102 receiving this RF-signal with high voltage in order to be operated using this signal.

When in operation the inverter circuit 114 is controlled such that it provides an RF-signal which will be having a voltage amplitude as high as needed at the generator 132. One value for controlling the signal at the generator output 132 is an output power $P_{OUT}$ which is supplied at the generator output 132 to the electro surgical instrument 102.

It is important that the output power $P_{OUT}$ corresponds to the corresponding set value of the output power. This output power is delivered to the electro surgical instrument and thus to the illustrated tissue 104 which is part of a human body. Providing too much power can be dangerous and is thus important that the provided output power is correct.

In order to ensure that there is a current sensor 140 and a voltage sensor 142. Based on these measured current and voltage signals a power calculation unit 144 calculates the corresponding output power $P_{OUT}$ the current sensor 140, voltage sensor 142 and power calculation unit 144 can be understood as forming an RF power acquisition device 146. However it is also possible to use the measured current signal and voltage signal and calculate the output power based on these signals in a section of the inverter control 128, or in a section which is part of another controller of the RF generator.

In any case such RF power acquisition device 146 is quite complex and costly. One reason is that the current sensor 140 and the voltage sensor 142 are measuring at a high voltage level and accordingly it is important to insulate these sensors from the connected power calculation unit 144, or any other calculation unit section be it a separate calculation unit or part of another controller.

As ensuring to supply the correct power is important there is often a redundancy necessary. Instead of measuring with a second device similar to RF power acquisition device 146 the output power directly, it is suggested to measure power at an input of the RF-generator 100. This is illustrated by the DC-sensor 150 which is also just illustrated schematically. Such DC-sensor 150 could be a current sensor measuring a DC-current. The DC-voltage at the supply output 110 could also be measured. Based on that the input power $P_{IN}$ can be calculated as an input power of the RF-generator. However it could also be assumed that the voltage amplitude at the intermediate circuit is constant or known by the inverter controller 128.

In any case such input power $P_{IN}$ at the input of the inverter can be measured much easier, i.e. with less expensive sensor than the output power $P_{OUT}$ is measured with the RF power acquisition device 146.

However it was found that the input power $P_{IN}$ is higher than the output power $P_{OUT}$, due to a power dissipation of several elements. Accordingly it is suggested to determine such power dissipation and that is explained by way of example in figure 1. The inverter control 128 knows the control scheme of the four semiconductor switches 121 - 124 and also knows a degree of modulation of the inverter. As explained above the inverter controller may also have information on the temperature of the semiconductor switches 121 - 124 and can thus calculate the power dissipation of this semiconductor switches.

However it is also possible, based on the known control scheme of the inverter and thus of the semiconductor switches, and based on an ambient temperature, to determine the power dissipation of the semiconductor switches. In any of the explained cases the inverter controller 128 can calculate a dissipation power of the inverter $P_{DI}$. There is also a significant power dissipation to be expected to the RF-transformer 130. Considering such power is also illustrated in figure 1 and accordingly the RF-transformer 130 outputs a value of the power dissipation of the transformer $P_{DT}$. All power dissipation parts collected this way, according to the example in figure 1 the power dissipation of the inverter $P_{DI}$ and the power dissipation of the transformer $P_{DT}$, can be added up to an overall power dissipation $P_D$. This is done in the summation element 152. This is just for illustrative purposes and other power dissipation parts could be considered in addition or it could be possible that one of the mentioned power dissipation parts is that small that it could be neglected, or could be a fairly constant value which could be considered thus as a constant value.

However in the subtraction element 154 the overall dissipation power $P_D$ is subtracted from the input power $P_{IN}$ and the result is the comparative power $P_C$. Accordingly the comparative power $P_C$ is compared with the output power $P_{OUT}$ in the logic block 156. This logic block 156 could be a single separate block or it could also be implemented in one of the shown or other controllers.

In particular it could be implemented in a general control of the RF-generator, or in the inverter controller 128. In this logic block 156 it is checked whether the comparative power $P_C$ equals the output power $P_{OUT}$. However there is an uncertainty acceptable and thus in the logic block the comparison checks whether both power values are approximately identical. One way of doing this could be by subtracting one from the other and check whether the amplitude of the difference is below a predetermined tolerance value. Such predetermined tolerance value could be in the range of 5 % to 20 % of the output power or of a set power.

Accordingly in the logic block the result of the comparison is either NO or YES and accordingly if the result of the comparison is NO the generator can be stopped. Otherwise if the comparison is YES the generator can keep on operating.

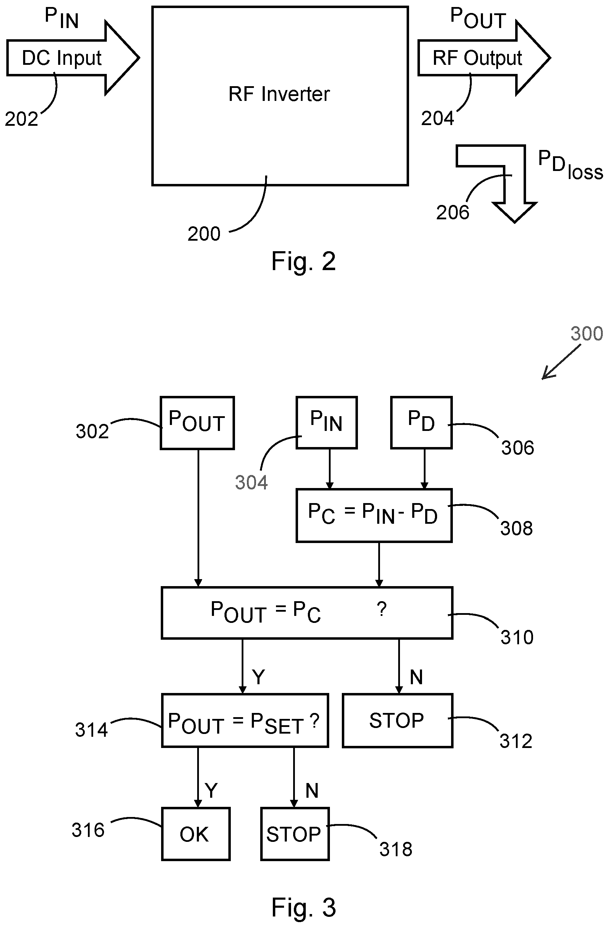

**Figure 2** briefly illustrates the underlying idea of the present invention. According to the diagram of figure 2 the RF-inverter 200 receives an input power $P_{IN}$ as an input signal 202. The result is an RF output signal 204 having an output power $P_{OUT}$. However the output power $P_{OUT}$ is smaller than the input power $P_{IN}$ as there is dissipation and that is illustrated by the overall dissipation power $P_D$. That is illustrated by a single dissipation signal 206. However there may be a plurality of elements or parts or places in the inverter which consume a part of the dissipation power.

The method according to one aspect is illustrated in the flow chart in **Figure 3** thus showing a method flow chart 300. At the beginning of the illustrated method the output power $P_{OUT}$ is recorded according to the output power recordal step 302. Meanwhile the input power $P_{IN}$ is recorded at the input of the RF-generator according to the input power recordal step 304 and there is also a power dissipation $P_D$ recordal according to the dissipation power recordal step 306.

The dissipation power or power dissipation will often not be a single power but a summation of various dissipation power parts as explained by way of example with respect to figure 1, illustrating the power dissipation of the inverter $P_{DI}$ and the power dissipation of the transformer $P_{DT}$.

In the subtraction step 308 the overall dissipation power $P_D$ is subtracted from the input power $P_{IN}$ and the result is the comparative power $P_C$.

In the comparison step 310 the output power $P_{OUT}$ and the comparative power $P_C$ are compared. This can be done by subtracting both values from each other and checking whether the result is, according to its absolute value, smaller than a predefindable threshold value. In that case the comparison would be true or YES, as there is only a small difference i.e. smaller than the mentioned threshold value.

According to the comparison at the comparison step 310 both values are not the same, i.e. the difference is higher than the just before mentioned threshold value, the power generator will be stopped immediately according to the first stop step 312.

If the comparison is positive, i.e. the output power $P_{OUT}$ and the comparative power $P_C$ are similar, then the result is YES and it may according to the value check step 314 further be checked, if the output power is also similar to a set power $P_{SET}$. The comparison can be performed similar as explained with respect to the comparison step 310. Accordingly it is checked whether not only both evaluated power values, i.e. the output power $P_{OUT}$ and the comperative power $P_C$ are similar, but also if the values are in the amount as wanted i.e. as set by the actual control of the electro surgical instrument. Accordingly if the result of the value check step is YES, everything is okay according to the confirmation step 316 and thus the RF-generator and thus the electro surgical instrument may keep on operating.

On the other hand if the result of the value check step is negative, then the generator will be stopped according to the second stop step 318.

According to the invention the following problem was identified. To ensure the correct/adjusted output of the RF power, current and voltage, the monitors for the RF voltage and the RF current are redundant. Thus, the output parameters are determined and compared in almost the same procedure.

In the event of a difference between the primary and redundant RF monitors in terms of measured RF current and/or RF voltage, a fault condition exists.

By executing the measurement method and/or using the system identically to determine the output parameters (this refers to both hardware, FPGA code and software, if an FPGA is used), design errors in both paths can lead to the same misbehaviour. Furthermore, the execution of an RF current and voltage measurement is complex and costly. Furthermore, additional sensors in the application circuit cause higher leakage currents, which must be minimized.

Accordingly known system may have the following disadvantages.

Known systems might be prone to design and systematic errors.

According to known solutions, there are also high costs, since the measuring system has to be implemented twice.

There might be higher leakage currents, because of additional bridging of the insulating distance by the sensors.

Based on that the following Idea and solution is suggested.

The verification of the output parameters should be carried out indirectly via the inverter input parameters as well as known losses. The input DC power (current, voltage) is determined and set in relation to the RF output power, taking into account losses of the RF inverter, e.g. thermal, electrical losses. That is illustrated by the following equation:

$$P_{HF-Output} = P_{DC-Input} - P_{Loss}$$

The power dissipation depends on various factors such as the modulation level of the RF, the RF mode, the ambient temperature, etc.. These factors are determined during development and are included in the calculation.

If there is a deviation between the calculated output power (can be named PHF output) and the actual output power (calculated from the RF output parameters), there is an error case (e.g. defective voltage sensor) and the system can react to this.

The proposed solution has the following advantages.

A costly design of RF current-voltage sensors (faster analog-digital-conversion or converters (ADC) and field programmable gate array (FPGA) and additional microcontroller unit (MCU) can be avoided.

A Lower leakage current can be achieved, as fewer sensors need to bridge the application circuit and secondary circuit.

**Claims**

1. Method for verifying a power recording of an RF generator to control at least one electrosurgical instrument, wherein

   - the RF generator has an inverter for generating an RF signal, and
   - the inverter

      - via an Inverter input is supplied with electrical current and
      - via an Inverter output emits an RF signal with a voltage value and a current value for driving an electrosurgical instrument,

comprising the steps

- recording of an output power delivered via the RF signal by directly measuring the RF signal by means of an RF power acquisition device, as directly recorded output power,
- recording of a comparative power based on a power measured on the input side of the inverter and
- verifying the functioning of the RF power acquisition device by comparing the recorded comparative power with the directly recorded output power.

2. The method according to claim 1, **characterized in that**

- on the Inverter input, an input power is recorded, and
- the comparative power is determined from the recorded input power minus a power dissipation.

3. The method according to claim 1 or 2, **characterized in that**

- the functioning of the RF power acquisition device is verified if an absolute value of a difference between the recorded comparative power and the directly recorded output power, is below a predetermined verification threshold value, wherein in particular
- the verification threshold value is in a range between 5% to 30% in particular 10% to 20% of the directly recorded output power.

4. A method according to any of the foregoing claims, **characterized in that**:

- a or the power dissipation is determined depending on at least one loss parameter, of the list comprising

- a degree of modulation of the inverter,
- a selected RF mode,
- an ambient temperature of the inverter,
- a component temperature of a component of the inverter,
- a switching frequency of the inverter, and
- a loss of a transformer.

5. A method according to any of the foregoing claims, **characterized in that**:

- at least one loss parameter

- recorded in advance, in particular before the inverter is put into operation, and assigned to a power dissipation, and/or
- is recorded during operation of the inverter and assigned to a power dissipation.

6. A method according to any of the foregoing claims, **characterized in that**:

- the inverter has a DC voltage input, and
- to record the comparative power, one or the input power from a DC voltage and/or DC current measurement at the Inverter input is detected.

7. A method according to any of the foregoing claims, **characterized in that**:

- the RF power acquisition device comprises an FPGA connected via an isolating transformer to the inverter output in order to measure the RF signal, and/or
- for measuring the power on the input side of the inverter a DC-measuring device is used.

8. A method according to any of the foregoing claims, **characterized in that**: a model is used

- for determining the comparative power and/or
- for determining the power dissipation.

9. A method according to any of the foregoing claims, **characterized in that**:

- an adaptive and/or self-learning estimation method and/or estimation model having estimation parameters, is used to estimate the comparative power, wherein in particular

- the comparative power is estimated based on initially set or previously adapted values of the estimation parameters,
- the estimated comparative power is compared with the directly recorded output power,
- if depending on the result of the comparison the functioning of the RF generator is verified,
- the directly recorded output power is identified as verified output power and
- the estimation parameters are adapted to align the estimated comparative power to the verified output power.

10. RF generator to control at least one electrosurgical instrument, adapted to verifying a power recording of the RF generator,

- the RF generator has an inverter for generating an RF signal, and
- the inverter, when in operation,
- via an Inverter input is supplied with electrical current and
- via an Inverter output emits an RF signal with a voltage value and a current value for driving the electrosurgical instrument,

and the RF generator is adapted to execute the steps of

- recording of an output power delivered via the RF signal by directly measuring the RF signal by means of an RF power acquisition device, as directly recorded output power,
- recording of a comparative power based on a power measured on the input side of the inverter and
- verify the functioning of the RF power acquisition device by comparing the recorded comparative power with the directly recorded output power.

**11.** RF generator according to claim 10, wherein

- the RF generator comprises an RF controller for executing and/or controlling the steps for verifying the power recording, and wherein
- the RF generator, in particular the RF controller is adapted to perform a method according to any of claims 1 to 9.

**11**

Fig. 1

$P_{IN}$

DC Input

202

RF Inverter

200

$P_{OUT}$

RF Output

204

$PD_{loss}$

206

## Fig. 2

300

302 — $P_{OUT}$

$P_{IN}$

304

$P_D$ — 306

$P_C = P_{IN} - P_D$ — 308

$P_{OUT} = P_C$ ? — 310

Y

N

314 — $P_{OUT} = P_{SET}$ ?

STOP — 312

Y

N

316 — OK

STOP — 318

## Fig. 3

Europäisches
Patentamt

European
Patent Office

Office européen
des brevets

# EUROPEAN SEARCH REPORT

**Application Number**

EP 25 16 0538

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/186959 A1 (COVIDIEN LP [US]) 9 September 2022 (2022-09-09) * paragraphs [0007], [0052] - [0056] * ----- | 1-11 | INV. A61B18/12 ADD. |
| A | US 2023/069525 A1 (DIJKSTRA JELLE [DE] ET AL) 2 March 2023 (2023-03-02) * paragraph [0082]; figure 10 * ----- | 1-11 | A61B18/00 |
| A | US 2008/082095 A1 (SHORES RONALD B [US] ET AL) 3 April 2008 (2008-04-03) * paragraphs [0094] - [0096] * ----- | 1-11 | |
| A | US 2023/397945 A1 (MAZUMDER SUDIP [US] ET AL) 14 December 2023 (2023-12-14) * paragraph [0198] * ----- | 1-11 | |

**TECHNICAL FIELDS
SEARCHED      (IPC)**

A61B

The present search report has been drawn up for all claims

| Place of search | Date of completion of the search | Examiner |
|---|---|---|
| Munich | 8 July 2025 | Aronsson, Fredrik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 16 0538

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2025

| Patent document cited in search report | Publication date | Patent family member(s) | | Publication date |
|---|---|---|---|---|
| WO 2022186959 A1 | 09-09-2022 | CN | 116916842 A | 20-10-2023 |
| | | EP | 4301261 A1 | 10-01-2024 |
| | | US | 2024122638 A1 | 18-04-2024 |
| | | WO | 2022186959 A1 | 09-09-2022 |
| US 2023069525 A1 | 02-03-2023 | CN | 115721405 A | 03-03-2023 |
| | | DE | 102021122282 A1 | 02-03-2023 |
| | | EP | 4140426 A1 | 01-03-2023 |
| | | EP | 4424256 A2 | 04-09-2024 |
| | | ES | 2995164 T3 | 07-02-2025 |
| | | JP | 7600189 B2 | 16-12-2024 |
| | | JP | 2023033229 A | 09-03-2023 |
| | | JP | 2025003944 A | 14-01-2025 |
| | | US | 2023069525 A1 | 02-03-2023 |
| US 2008082095 A1 | 03-04-2008 | AU | 2006228041 A1 | 17-04-2008 |
| | | CA | 2563484 A1 | 02-04-2008 |
| | | GB | 2442560 A | 09-04-2008 |
| | | GB | 2448585 A | 22-10-2008 |
| | | JP | 6174987 B2 | 02-08-2017 |
| | | JP | 2008086776 A | 17-04-2008 |
| | | JP | 2014100583 A | 05-06-2014 |
| | | US | 2008082095 A1 | 03-04-2008 |
| US 2023397945 A1 | 14-12-2023 | NONE | | |

EPO FORM P0459

For more details about this annex : see Official Journal of the European Patent Office, No. 12/82